# EUROPEAN PATENT SPECIFICATION

(11) **EP 1 761 254 B1**
(45) Date of publication and mention of the grant of the patent: **02.09.2009**
(21) Application number: 05759460.8
(22) Date of filing: 08.06.2005
(51) Int. Cl.: A61K 31/07, A61P 27/02

(54) **IMPROVEMENT OF IMAGE QUALITY IN THE EYE**
VERBESSERUNG DER BILDQUALITÄT IM AUGE
AMELIORATION DE LA QUALITE DE L'IMAGE DANS L'OEIL

(30) Priority: 29.06.2004 EP 04015189
(43) Date of publication of application: 14.03.2007
(73) Proprietor: DSM IP Assets B.V., 6411 TE Heerlen (NL)
(72) Inventor: BARBUR, John, Berkhamsted, Herts HP4 3LZ (GB)
(74) Representative: Steck, Melanie
(86) International application number: PCT/EP2005/006168
(87) International publication number: WO 2006/002735

(56) References cited:
- WO-A-97/48388
- WO-A-03/063848
- SCHALCH, WOLFGANG: "Lutein and zeaxanthin , the xanthophylls of the macula lutea" AGRO FOOD INDUSTRY HI-TECH , 14(2), 23-27 CODEN: AIHTEI; ISSN: 1722-6996, 2003, XP008052683
- ALVES-RODRIGUES, ALEXANDRA ET AL: "The science behind lutein" TOXICOLOGY LETTERS, vol. 150, no. 1, 2004, pages 57-83, XP002345432
- DATABASE WPI Section Ch, Week 200439 Derwent Publications Ltd., London, GB; Class B05, AN 2004-412176 XP002345434 & CN 1 481 804 A (WUXI JIEXI MEDICINE TECHNOLOGY LTD) 17 March 2004 (2004-03-17)
- KVANSAKUL ET AL: 'Supplementation with the carotenoids lutein or zeaxanthin improves human visual performance' OPHTHAL. PHYSIOL. OPT. vol. 26, 2006, pages 362 - 371

## Description

The present invention relates to the improvement of image quality in the eye. The term "image quality" as used herein refers to the image formed on the retina. This image can be deteriorated by higher-order aberrations such as spherical aberrations, coma and irregular astigmatism.

While lower-order aberrations limit how much we can see, higher-order aberrations determine how well we can see. Higher-order aberrations are aberrations of the optics of the eye above and beyond nearsightedness, farsightedness and astigmatism. Higher-order aberrations cannot be corrected easily with glasses or contact lenses. Higher-order wavefront aberrations describe small deviations of the wavefront surface from the ideal surface and the reproduction of these deviations require a large number of components. Two common and potentially disruptive higher order aberrations are "spherical aberration" and "coma" see Liang J, Williams DR; Aberrations and retinal image quality of the normal human eye; J. Opt. Soc. Am. A. 1997;14 (11) :2873-83). For the visual benefits of correcting higher-order aberrations of the eye see also Williams et al., Visual Benefit of Correcting Higher Order Aberrations of the Eye, J. Refract. Surg. 2001,16 (Sept./Oct. 2000) S554 - S559.

The published international patent application WO03/063848 discloses an improvement of visual performance, particularly of visual performance in the darkness, by administration of a colorant that is capable of being incorporated into eye tissue and/or causing yellowing eye tissue, especially carotenoids, such as lutein and zeaxanthin.

In accordance with the present invention it has been found that the administration of lutein and/or zeaxanthin leads to a reduction of higher-order wavefront aberrations in the eye. Thus, in one aspect, the invention relates to the use of lutein or zeaxanthin, or mixtures thereof, in the manufacture of a composition for reducing higher-order wavefront aberrations in the human eye.

The term "higher order wavefront aberrations in the eye" as used herein denotes aberrations of the optical system of the eye that are distinct from myopia, hyperopia and axial astigmatism but nevertheless influence the quality of the image formed on the retina. They are measured by commercially available apparatuses, and are expressed as the wavefront function, which completely describes the aggregate effects of all refractive parts of the eye on light passing through every location of the pupil.

The wavefront aberration function is a way of quantifying the imperfections of the optics of the eye that cause degradation of retinal image quality. The "ideal" wavefront is generated when a "point" on the retina is imaged through the optics of the eye as a plane and flat surface just outside the eye. This means that all the rays emerging from the eye form a parallel bundle and are perpendicular to this plane. The "real" wavefront surface is not a perfect two-dimensional plane and reflects the aberrations present in the optical system. It is usually represented mathematically by a series of terms known as Zernike polynomials. A knowledge of the coefficients of the Zernike polynomials provides a complete description of the wavefront surface. In order to obtain one single number that describes the quality of the imaging system (in this case the human eye) the root mean square (rms) deviation is calculated by computing the root of the mean of the squares of the actual deviations from the ideal plane. The rms value is equivalent to a standard deviation, but the two functions compared are 2D surfaces (i.e., the ideal wavefront and the actual wavefront). The wavefront aberrations are measured with a wavefront analyzer (see, e.g., J. Refract. Surg. 2001; 17(5), S608-S612). The rms wavefront aberration increases significantly with the diameter of the pupil and can show large inter-subject variability. A small wavefront aberration value means better image quality on the retina and this translates to improved visual performance, (e.g., ability to see smaller or very faint objects, sharper and crisp images).

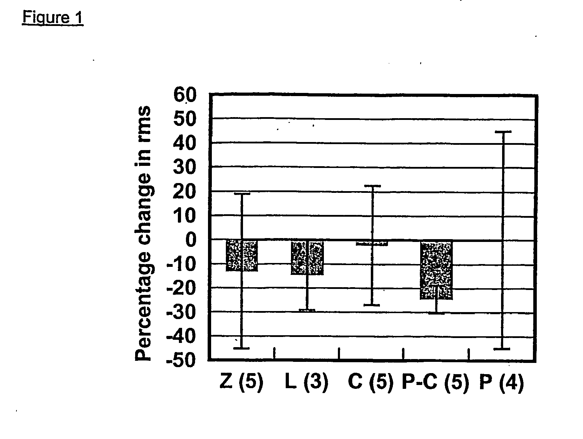

The reduction of higher-order wavefront aberrations in the human eye on administration of lutein and/or zeaxanthin can be seen from Figure 1.

In Figure 1 the number in parentheses is the number of subjects treated for 6 months with 20 mg/day zeaxanthin (Z), 20 mg/day lutein (L), 10 mg lutein + 10mg zeaxanthin per day (C , P-C), or placebo (P).

The term "composition" as used herein denotes any composition that is suitable for administration to the human body, such as pharmaceutical preparations or dietary supplements in dosage unit form, or a food, or a beverage.

A pharmaceutical preparation or dietary supplement in accordance with the present invention may be in any form that is conventional for oral administration, e.g. in solid form such as tablets including effervescent tablets, or soft or hard shell capsules, or in liquid form, such as solutions or suspensions, preferably oily suspension. Besides the active ingredients these preparations may contain conventional carrier material, additives and adjuvants, which include water, gelatin, vegetable gums, sugars, vegetable oils, polyalkylene glycols, flavoring agents, preservatives, stabilizers, emulsifying agents, and buffers. The medicaments may be in the form of controlled (delayed) release formulations. For the purpose of the invention the colorants as well as optional ingredients as defined earlier hereinabove may be incorporated in food or beverages, such as bakery items, e.g., cake and cookies, lemonades and fruit juices. The compositions according to the present invention may further contain physiologically active ingredients conventionally used to promote health, especially eye health, such as vitamins e.g. vitamin A, C and E, and minerals, such as selenium or zinc.

A suitable daily dosage of lutein and/or zeaxanthin for the purposes of the present invention may be within the range from 0.001 mg per kg body weight to 20 mg per kg body weight. More preferred is a daily dosage of 0.01 to 10 mg per kg body weight, and especially preferred is 0.1 to 1.0 mg per kg body weight per day.

In a preferred aspect, the invention relates to the use of a combination of lutein and zeaxanthin. In such combination these compounds are preferably used in a ratio of 0.1-1.0 : 1.0- 0.1 parts by weight.

In solid dosage unit preparations, lutein and/or zeaxanthin are suitably present in an amount from 0.1 mg to 500 mg, preferably from 1 mg to 100 mg per dosage unit. In liquid formulations, the aforesaid ingredients are suitably present in an amount of from 0.1 to 5 percent by weight based upon the total weight of the composition.

Preferred solid dosage unit preparations comprise, per dosage unit, 6 mg to 12 mg of lutein and/or zeaxanthin.

The invention is illustrated further by the Examples given below:

### Example 1

A soft gelatin capsule may be prepared comprising the following ingredients:

| Ingredient | Amount per Capsule |
|---|---|
| Lutein | 10 mg |
| Lecithin | 50 mg |
| Soy bean oil | 200 mg |

### Example 2

A soft gelatin capsule may be prepared comprising the following ingredients:

| Ingredient | Amount per Capsule |
|---|---|
| Lutein | 10 mg |
| Zeaxanthin | 10 mg |
| Lecithin | 50 mg |
| Soy bean oil | 200 mg |

### Example 3

A soft gelatin capsule may be prepared comprising the following ingredients:

| Ingredient | Amount per Capsule |
|---|---|
| Lutein | 6 mg |
| Zeaxanthin | 6 mg |
| Vitamin E (α-d,l-tocopherol) | 200 mg |
| Vitamin C | 500 mg |
| Lecithin | 50 mg |
| Soy bean oil | 200 mg |

### Example 4

A soft gelatin capsule may be prepared comprising the following ingredients:

| Ingredient | Amount per Capsule |
|---|---|
| Lutein | 12 mg |
| Vitamin E (α-d,l-tocopherol) | 200 mg |
| Vitamin C | 500 mg |
| Lecithin | 50 mg |
| Soy bean oil | 200 mg |

### Example 5

A soft gelatin capsule may be prepared comprising the following ingredients:

| Ingredient | Amount per Capsule |
|---|---|
| Zeaxanthin | 12 mg |
| Vitamin E (α-d,l-tocopherol) | 200 mg |
| Vitamin C | 500 mg |
| Lecithin | 50 mg |
| Soy bean oil | 200 mg |

### Example 6

A soft gelatin capsule may be prepared comprising the following ingredients:

| Ingredient | Amount per Capsule |
|---|---|
| Lutein | 6 mg |
| Zeaxanthin | 6 mg |
| β-Carotene | 6 mg |
| Vitamin E (α-d,l-tocopherol) | 200 mg |
| Vitamin C | 500 mg |
| Zinc (as orotate) | 7.5 mg |
| Lecithin | 50 mg |
| Soy bean oil | 200 |

### Example 7

A soft gelatin capsule may be prepared comprising the following ingredients:

| Ingredient | Amount per Capsule |
|---|---|
| Lutein | 6 mg |
| Zeaxanthin | 6 mg |
| Vitamin E (α-d,l-tocopherol) | 200 mg |
| Vitamin C | 500 mg |
| Vitamin A | 1000 Int. Units |
| Zinc (as orotate) | 7.5 mg |
| Lecithin | 50 mg |
| Soy bean oil | 200 mg |

### Example 8

A soft gelatin capsule may be prepared comprising the following ingredients:

| Ingredient | Amount per Capsule |
|---|---|
| Lutein | 6 mg |
| Zeaxanthin | 6 mg |
| β-Carotene | 6 mg |
| Vitamin E (α-d,l-tocopherol) | 200 mg |
| Vitamin C | 500 mg |
| Vitamin A | 1000 Int. Units |
| Zinc (as orotate) | 7.5 mg |
| Lecithin | 50 mg |
| Soy bean oil | 200 mg. |

## Claims

1. The use of lutein or zeaxanthin, or mixtures thereof in the manufacture of a composition for reducing higher order wavefront aberrations in the human eye to improve image quality.

2. The use of lutein according to claim 1.

3. The use zeaxanthin according to claim 1.

4. The use according to any one of claims 1-3 wherein the composition is a pharmaceutical composition or dietary supplement in dosage unit form.

5. The use according to claim 4 wherein the composition is for oral application and contains per dosage unit an amount of 0.1 mg to 600 mg of lutein or zeaxanthin, or mixtures thereof.

6. The use according to any one of claims 1-3 wherein the composition is a food or beverage.

7. The use according to claim 1 wherein the composition is to be administered at a daily dosage of lutein or zeaxanthin, or mixtures thereof within the range of from 0.001 mg per kg body weight to 20 mg per kg body weight.

8. The use according to claim 7 wherein the composition is to be administered at a daily dosage of lutein or zeaxanthin, or mixtures thereof within the range of from 0.01 to 10 mg per kg body weight.

9. The use according to claim 8 wherein the composition is to be administered at a daily dosage of lutein or zeaxanthin, or mixtures thereof within the range of from 0.1 to 1.0 mg per kg body weight per day.

## Patentansprüche

1. Verwendung von Lutein oder Zeaxanthin oder Mischungen davon bei der Herstellung einer Zusammensetzung zum Reduzieren von Wellenfrontaberrationen höherer Ordnung im menschlichen Auge, um die Bildqualität zu verbessern.

2. Verwendung von Lutein nach Anspruch 1.

3. Verwendung von Zeaxanthin nach Anspruch 1.

4. Verwendung nach einem der Ansprüche 1-3, wobei die Zusammensetzung eine pharmazeutische Zusammensetzung oder ein Nahrungsergänzungsmittel in Dosiseinheitsform ist.

5. Verwendung nach Anspruch 4, wobei die Zusammensetzung zur oralen Verabreichung bestimmt ist und pro Dosiseinheit eine Menge von 0,1 mg bis 500 mg Lutein oder Zeaxanthin oder Mischungen davon enthält.

6. Verwendung nach einem der Ansprüche 1-3, wobei die Zusammensetzung ein Lebensmittel oder ein Getränk ist.

7. Verwendung nach Anspruch 1, wobei die Zusammensetzung mit einer Tagesdosis von Lutein oder Zeaxanthin oder Mischungen davon innerhalb des Bereichs von 0,001 mg pro kg Körpergewicht bis 20 mg pro kg Körpergewicht verabreicht wird.

8. Verwendung nach Anspruch 7, wobei die Zusammensetzung mit einer Tagesdosis von Lutein oder Zeaxanthin oder Mischungen davon innerhalb des Bereichs von 0,01 bis 10 mg pro kg Körpergewicht verabreicht wird.

9. Verwendung nach Anspruch 8, wobei die Zusammensetzung mit einer Tagesdosis von Lutein oder Zeaxanthin oder Mischungen davon innerhalb des Bereichs von 0,1 bis 1,0 mg pro kg Körpergewicht verabreicht wird.

## Revendications

1. Utilisation de la lutéine ou de la zéaxanthine, ou des mélanges de celles-ci, dans la fabrication d'une composition destinée à la réduction des aberrations du front d'onde d'ordre supérieur dans l'oeil humain pour améliorer la qualité d'image.

2. Utilisation de la lutéine selon la revendication 1.

3. Utilisation de la zéaxanthine selon la revendication 1.

4. Utilisation selon l'une quelconque des revendications 1 à 3, **caractérisée en ce que** la composition est une composition pharmaceutique ou un complément alimentaire en forme de dose unitaire.

5. Utilisation selon la revendication 4, **caractérisée en ce que** la composition est destinée à l'application par voie orale et contient par dose unitaire une quantité de 0,1 mg à 500 mg de lutéine ou de zéaxanthine, ou des mélanges de celles-ci.

6. Utilisation selon l'une quelconque des revendications 1 à 3, **caractérisée en ce que** la composition est un aliment ou une boisson.

7. Utilisation selon la revendication 1, **caractérisée en ce que** la composition doit être administrée à une dose journalière de lutéine ou de zéaxanthine, ou de mélanges de celles-ci, dans la gamme de 0,001 mg par kg de poids corporel à 20 mg par kg de poids corporel.

8. Utilisation selon la revendication 7, **caractérisée en ce que** la composition doit être administrée à une dose journalière de lutéine ou de zéaxanthine, ou de mélanges de celles-ci, dans la gamme de 0,01 à 10 mg par kg de poids corporel.

9. Utilisation selon la revendication 8, **caractérisée en ce que** la composition doit être administrée à une dose journalière de lutéine ou de zéaxanthine, ou de mélanges de celles-ci, dans la gamme de 0,1 à 1,0 mg par kg de poids corporel par jour.
